(19)

Europäisches Patentamt

European Patent Office

Office européen des brevets

(11) **EP 3 114 991 A1**

(12) **EUROPEAN PATENT APPLICATION**

(43) Date of publication:
**11.01.2017 Bulletin 2017/02**

(21) Application number: **16000336.4**

(22) Date of filing: **09.02.2016**

(51) Int Cl.:
*A61B 5/04* (2006.01)          *A61B 5/0452* (2006.01)
*A61B 5/02* (2006.01)          *A61B 5/00* (2006.01)
*A61B 5/0408* (2006.01)          *G06F 19/00* (2011.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **10.07.2015 EP 15002065
20.07.2015 EP 15002150
24.01.2016 EP 16000163**

(71) Applicant: **Alekseev Alekseev Siegle Röder GbR
81479 München (DE)**

(72) Inventors:
• **Alekseev, Mikhail
St. Petersburg 197755 (RU)**
• **Alekseev, Aleksandr
St. Petersburg 197755 (RU)**
• **Röder, Gunther
81479 München (DE)**
• **Siegle, Reinhard
86153 Augsburg (DE)**

(54) **DATA PROCESSING APPARATUS FOR MYOCARDIUM CONDITION ASSESSMENT**

(57)     The invention concerns a data processing apparatus (12) for processing T-wave information (TI) or ST-segment (ST) information from an electrical signal from a myocardium of a heart of a human or animal, for an assessment of a condition of at least a part of the myocardium or the whole myocardium, characterised in that the data processing apparatus (12) is configured to derive a T-wave deviation value (DV, NDV, 2DV, N2D) from a difference of at least two T-wave parameters (P) or ST-segment parameters (P), wherein each of the two parameters (P) belongs to another T-wave (T) or ST-segment (ST) from the same heart. Further, an assessment apparatus for assessing the condition of at least a part of a myocardium of a heart of an individual human or animal, a method for providing data for an assessment of a condition of at least a part of a myocardium of a heart of a human or an animal, wherein the method includes processing T-wave information from an electrical signal from at least a part of a myocardium and a diagnosis method for diagnosing a condition or a disease of a heart are proposed. Further, a method for data processing, a method for assessing a condition or a disease of the heart and a diagnosis method are proposed.

Fig. 1

EP 3 114 991 A1

**Description**

This invention concerns a data processing apparatus for processing T-wave or ST-segment information from an electrical signal from a myocardium of a heart of a human or animal, for an assessment of a condition of at least a part of the myocardium or the whole myocardium. Further, an assessment apparatus for assessing the condition of at least a part of a myocardium of a heart of an individual human or animal is provided. Furthermore, a method for providing data for an assessment of a condition of at least a part of a myocardium of a heart of a human or an animal, wherein the method includes processing T-wave or ST-segment information from an electrical signal from at least a part of a myocardium and a diagnosis method for diagnosing a condition or a disease of a heart are provided.

Description

State of the art

It is common knowledge that a myocardial infarct can be the terminal stage of ischemic heart disease which means a regionally reduced blood flow in the myocardium. This can be a result of an arterioscleortic disease of an epicardial coronary artery which can lead to a stenosis, of a microvascular dysfunction and of a vasospasm. In this way, perfusion of the myocardium is regionally impaired which leads to a shortage in supply of this region. In order to diagnose ischemic heart disease, several methods are used, including anamnesis, biochemical tests, echocardiography, invasive coronary angiography which is considered a gold standard, coronary computer tomography angiography, magnetic resonance imaging angiography and electrocardiography.

Emergency department clinicians have a difficult task in identifying which patients to admit to hospital and which patients to discharge home. Of the patients presenting to the emergency department for chest pain, 55 to 85 percent do not have a cardiac cause for their symptoms. Of those admitted for chest pain, more than 60 percent do not have acute coronary syndromes. Unnecessary admissions for a chest pain in the U.S. alone cost billions of dollars annually.

In a study of over 15.000 emergency department chest pain patients, it was found that 48 percent had a final emergency department diagnosis of chest pain not otherwise specified. It is a disadvantage that no definitive diagnosis could be made in such a large percentage of these patients, some of whom may have had an acute coronary syndrome. Missed diagnosis is associated with twofold increased mortality risk. The rate is unacceptable from a patient perspective and from a healthcare perspective as well.

In the U.S., about 5.8 million patients visit the emergency department (ED) for chest pain and related symptoms, and about 4.4 million for chest pain alone. As many as 79 percent of U.S. chest pain patients who visit the emergency department are low-risk. At discharge, it is estimated that as many as 85 percent of chest pain patients do not have a cardiac diagnosis, and for 2 to 8 percent, the diagnosis was missed.

In a typical case, a patient with angina pectoris as a first symptom is hospitalized or treated by an emergency doctor. In a first step, common electrocardiography is carried out. In some cases, an ischemic heart disease can be diagnosed by an elevation of the ST segment at this stage. But a significant portion of the patients do not exhibit this symptom though they suffer from ischemic heart disease, as can turn out later. For these patients, there is the possibility to carry out laboratory diagnostics on troponine level or on a level of other biochemical markers which are elevated from ischemic heart disease. This can only be done after a few hours from the start of angina pectoris on, what is too late for many patients. Also, the other known methods except echocardiography and anamnesis need time for preparation and cannot be carried out immediately. This is a significant disadvantage of the state of the art.

It is also known that there are patients that suffer a myocardial infarction even without any symptoms of ischemic heart disease, and also the myocardial infarction can be with normal coronary arteries or without significant stenosis (syndrome X). Therefore, the ischemic heart disease cannot be diagnosed by angiography or echocardiography. Therefore, there are cases in which ischemic heart disease cannot be diagnosed at all and which can lead to myocardial infarction without symptoms which are, according to the state of the art, detectable in advance. This can be a reason for a sudden heart death. The lack of diagnosis methods is also is a severe disadvantage of currently used diagnosis methods.

Further, according to the state of the art, it is difficult to estimate a severity of the ischemic heart disease and impossible to obtain it with an immediately applicable method.

In the state of the art, the measurement of T-wave alternans is known. With this method, the ventricular arrhythmia and sudden heart death can be prognosed with a certain probability. T-wave alternans has also proven to be useful for determining implantation of implantable cardiac defibrillators. In an electrocardiogram measurement, an alternation in the morphology of T-wave occurs in every second beat, such that a similar form of T-wave occurs at each second beat. By a T-wave alternans measurement, the difference of amplitudes of the first and the second T-waves is determined as a result. This can take place with the precision of a few microvolts. For improvement of precision, a spectral method (Cohen and Smith, M.I.T) and a modified moving average method (Nearing and Verrier, Harvard Medical School) can be used.

Anarticle by Alekseyev, Michael; Alekseyev Aleksandr; Dowzhikov, Andrew and Labin, Sergei: "Digital Analytic Cardiography (DACG), a New Method for Quantitiative Trophism Assessment of the Myorcardium". International Journal of Cardiovascular Research, 2014, 3:6 discloses clinical measurements of quantitative criteria G, L and S for improved diagnostics of ischemic heart disease. It shall be included in this patent application by reference concerning its parts that deal with measurement, processing and interpretation of electrocardiography data and data which have been derived thereof.

Objective of this invention is to provide an apparatus and a method for data processing with the goal of assessing a condition or a disease of a myocardium or a part of it. Assessment data as a basis for the assessment shall be provided by a data processing apparatus and/or a data processing method.

Subject matter of the invention is a data processing apparatus for generating assessment data for assessment of a condition of a myocardium or a part of a myocardium. The apparatus processes T-wave information or ST-segment information of at least two T-waves or ST-segments which are commonly known as a part of a PQRST-complex of an electrocardiogram.

T-wave information as well as ST-segment information comprises all information, that can be derived from the respective part of the waveform. Particularly, a time period and/or an amplitude are relevant.

It has been discovered that the same information is present in T-wave and in ST-segment. The relevant information can generally be extracted in the same way from the measured raw information of T-wave and ST-segment. Particularly, there is the same information in temporal deviations of a time period of ST-segment as in temporal deviations of a time period of T-wave. Particularly, there is also the same information in temporal deviations of the amplitude of T-wave as well as temporal deviations of the amplitude level of ST-segment. In the following, if only T-waves, T-wave information and T-wave values derived from T-wave information are mentioned, the applicants declare that also ST-segments, ST-segment information and ST-segment values shall be meant and comprised when T-wave information or T-wave values are mentioned, respectively, except something different is indicated explicitly.

It has been discovered that there is a correlation between temporal deviations of several parameters of T-wave and the condition of the myocardium that delivers the T-wave which can be correlated to a condition of the myocardium or a part of it. A "condition" of a myocardium or a part of it or of a heart means in this context a presence of one or more certain metabolisms or grade(s) of activity of one or more certain metabolisms, respectively. A "condition" also means certain pathological or non-pathological states of the myocardium or a part of the myocardium providing the T-wave of the myocardium. The state of the myocardium and, in consequence, of the heart is considered a result of the metabolism. As a result from a clinical study it seems clear that a myocardial metabolism is in close correlation with ischemic heart disease. Thus, both a metabolism and its results, namely different heart diseases can be detected. The results of corresponding clinical studies have been published by Michael Alekseyev, Aleksandr Alekseyev, Andrew Dowzhikov and Sergei Labin as mentioned above.

The inventors have found out that an ischemic heart disease does not only rely on the amount of blood supply to the myocardium which can be reduced by a stenosis, for example, but, with a better correlation, relies on a state of metabolism of the myocardium which is inter alia a result of blood supply. It is known that the T-wave represents the process of repolarisation of the myocardium. In dependency from the condition, the regularity of recovery is different such that the deviation value represents the condition of the process of repolarisation. Also, the state of depolarised ventricles which is represented by the ST-segment is affected by ischemic heart disease in a very similar way as the repolarisation process represented by T-wave.

An apparatus according to the invention derives at least one T-wave deviation value or at least one ST-segment deviation information from information of at least two T-waves or ST-segments, respectively, from a heart of a human or animal, the T-waves or ST-segments having occurred and preferably been measured at different times. The T-waves or ST-segments can also be derived from the same heart beat but from different locations. Preferably, this is done with measured data from an individual human or animal, preferably during a defined period of time or from a defined number of heartbeats.

The assessment data are based on differences between at least two parameters of T-wave or ST-segment. The differences have a good correlation to the myocardial condition and the ischemic heart disease.

"Individual human or animal" means in this context one single human or animal. A "deviation" means in this context that at least parts of the information of the T-waves or ST-segments change between single measurements of T-wave. Significant parts of the information, for example a time period of T-wave of ST-segment or a defined part of it, a maximum amplitude of T-wave or a defined part of it or an amplitude level of ST-segment, or an area which is bordered by a T-wave or ST-segment or a defined part of it, or function parameters of a mathematical function which matches or approximates a T-wave or ST-segment are called a "parameter" in this patent application. Different parameters are defined in the subclaims. A "difference" does not necessarily mean a mathematical difference in this context, but a mathematical difference might of course be used for derivation of a T-wave deviation value from parameters. Alternatively a comparison of the parameters can be used to assign a certain comparison result to the T-wave deviation value or ST-segment deviation value or a lookup table or another known method for deriving a deviation might be used. If more than two parameters of single heart beats are used for derivation of one or more T-wave deviation values or ST-segment deviation values, also statistical methods can be used. A deviation can be calculated for example as a statistical standard deviation by using a standard deviation calculation algorithm as known in statistics or an equivalent deviation calculation method known from mathematics or statistics, for example calculation of a variance . Further, a discrete derivative with respect to time can be calculated to obtain information which is present in the changes between single parameters of single heart beats. For example a mean value or frequently appearing values or a difference between a minimum and a maximum or other representative values of the derivative can be taken as a T-wave deviation or ST-segment deviation value, respectively.

Into the apparatus, functions of an electrocardiograph can be integrated. The apparatus can also be independent from an electrocardiograph and can obtain the T-wave deviation value from T-wave information which is supplied to the apparatus.

Preferably, the T-wave information and/or ST-segment information is measured with a digital result. Measurement is not necessarily a part of the data processing device. Input data for the data processing device can also be supplied by a separate measurement unit or be taken from a data memory.

Preferably, the apparatus provides the T-wave deviation value and/or the ST-segment deviation value for assessment of the condition. This provision can for example be to a user, to an automated assessment apparatus or to a memory from where it can be retrieved later.

In embodiment of the data processing apparatus, the T-wave information for obtaining the parameters has a resolution of less than 5 ms, or 285$\mu$s, preferably less than 100$\mu$s and most preferable less than 50$\mu$s in time and of less than 50mV or 16$\mu$V in amplitude, preferably less than 1$\mu$V and most preferable less than 1 nV. The resolution values are meant to be effective resolution values which might also be achieved by using a worse real resolution and more data, whereof the relevant information can be retrieved by mathematical or statistical methods.

With the proposed amplitude resolution of the input data for the data processing apparatus of 50mV and a time resolution of 5ms, some useful results can be obtained. With an amplitude resolution of 16$\mu$V or better and/or a time resolution of 285$\mu$s or better, the deviations correlating with a myocardium condition can be detected in a better quality. The better the resolution, the more precise assessment data result. With an amplitude resolution of 1$\mu$V or better, good results can be achieved. With a resolution of 1 nV or better, very good results can be achieved. Also, with a time resolution of 100$\mu$s or better good results can be achieved, and with a time resolution of 50$\mu$s or better, very good results can be achieved. It can, however, be sufficient that only parts of the information that are used for obtaining T-wave parameters have sufficient resolution. In case that an analog-to-digital-converter is used for digitalisation of input data, it can have a relative resolution that enables the above mentioned absolute resolution at least for relevant T-wave information. An amplifier can be used to adapt the measurement range and the absolute resolution of an analog-to-digital-converter. A person skilled in the art can easily calculate the relative resolution and the number of bits (bit-width) of an analog-digital-converter for the measurement from the measurement range and the absolute resolution. The amplitude of the T-wave preferably fits into the measurement range. At least, the relevant parts of the T-wave fit into the measurement range. The QRS- complex does not necessarily have to fit in the measurement range completely for one example embodiment of this invention.

There are different metabolism types in a myocardium. A myocardium has an anaerobic part of its metabolism for supplying the myocardium with energy. The anaerobic metabolism can have two different metabolism paths. One of them is using lactate (L-metabolism) for energy production and the other one is using glucose (G-metabolism). It has been discovered that the percentage of stenosis and the L-metabolism are correlated with time period deviations of T-wave, respectively, and that the G-metabolism is correlated with amplitude deviations of T-wave. Therefore, the presence and an amount of the L-metabolism and of the G-metabolism and therefore of anaerobic metabolism can be detected by a T-wave deviation value with the parameters time period and amplitude of the T-wave.

In a further embodiment, the parameter is a time period of the T-wave or of the ST-segment or of a characteristic part of them, respectively. T-wave or ST-segment deviation values generated by this parameter can be provided and used for assessment of the activity and the grade of activity of a lactate metabolism of the myocardium. The detection of the beginning and the end of T-Wave and ST-segment, respectively, can be made according to standard methods of signal processing. In respect of recognising the beginning and the end of a time period, there can be differences between the treatment of T-wave information and ST-segment information. It can also be thought of using other significant points of T-wave for measuring a T-wave time period, such as a period between an intermediate point at a certain percentage of height on a slope of T-wave and measure the time between them. A measurement of a period between a percentage point and one end of T-wave is also possible. Also, a point of maximum or minimum gradient in regard of T-wave time period can be a characteristic point.

A characteristic part is a part of T-wave in time is a time period between characteristic points of T-wave. Also the beginning and the end of T-wave are characteristic points.

In regard of ST-segment, the beginning of the time period can be recognised by a maximum curvature at the transition from the rising part of S wave to ST segment. Correspondingly, the transition of ST-segment to T-wave can be recognised by a maximum curvature. Also, the beginning and the end can be recognised by an increasing deviation of the waveform from a line, preferably, as a point in time at which signal exceeds a certain difference from a line function which can be defined to represent ST-segment. The beginning of the ST-segment cannot be recognised as the top of a little intermediate peak which is located between the rising part of the waveform after the S-wave and ST-segment. Further, the beginning and the end of can be recognised as points in the waveform at which a certain gradient is present. These and other known methods known from mathematics or signal processing can be used to detect the beginning and the end of a line formed segment in the waveform is the beginning and the end of ST-segment. The time period of ST-segment does not necessarily have to be calculated between the beginning and the end of ST-segment, but can also be calculated between other significant points in ST-segment in case that they are present. This is possible because also in parts of ST-segment, the information is present.

In a further embodiment, the parameter is an amplitude of the T-wave or a characteristic part of it, or a level of ST-segment. T-wave deviation values or ST-segment deviation values generated by this parameter can be provided and used for assessment of the activity and the grade of activity of a glucose metabolism of the myocardium.

The amplitude of a T-wave can for example be calculated by subtracting a maximum value from a minimum value. It is also possible to use a voltage difference between significant points of T-wave. Significant points can for example be an isoline, an end of an ST-line, a line between T-wave and P-wave, a maximum value of T-wave and a point of T-wave after a certain time after its beginning as well as a point of a maximum gradient regarding time. Also, only the maximum or a certain percentage of it can be measured and used as the amplitude. This is possible, because T-wave normally starts and ends at zero where the voltage usually is about 0V. It is also possible that T-wave extends to negative voltage. Then the amount of the negative amplitude is used. T-waves with abnormal deviations which occur exceptionally can be omitted for derivation of a T-wave deviation value. This is true for all embodiments.

The level of the T-segment can for example be obtained by calculating an average value of the measurement values between the beginning and the end of ST-segment. This also possible to use one of the amplitudes at the beginning and the end point of ST-segment or a mean value thereof. Also, further methods known in mathematics or signal processing in order to determine a level of a line segment of the waveform can be used in order to obtain the level of ST-segment.

In a further embodiment, the parameter is an area which is partially bordered by the T-wave or by the ST-segment.. A further border of the area can be the isoline or a line parallel to the isoline. In case of ST-segment, also connection lines between the isoline or a line parallel to the isoline and ST-segment can form part of the board of the area. Preferably, the connection lines are arranged perpendicular to the isoline and/or to the ST-segment. Preferably, the connection lines can comprise the beginning point and/or the end point or other significant points of ST-segment. T-wave deviation values generated by this parameter can be provided and used for assessment of a combined grade of activity of a lactate metabolism and activity of a glucose metabolism of the myocardium. The parameter can represent anaerobic metabolism of the myocardium. The area can for example be calculated by integration or a summation of amplitude points along T-wave or ST-segment. For example, integration or summation can end at the isoline or another defined level of voltage. In case of ST-segment, also a multiplication of the time period by an amplitude is possible. An advantage of the parameter of an area is that the area is calculated from many single T-wave informations such that a comparably good accuracy of the parameter is achieved as signal noise has less effect. This parameter can be used in an apparatus which provides measurement of anaerobic metabolism. A less accurate measurement is required which can save costs.

In a further embodiment, the parameter is a function parameter of a function that approximates or matches the T-wave and/or the ST-segment. It is also possible to find an approximation function for both T-wave and ST-segment. For this, the waveform of T-wave and/or of the ST-segment is preferably measured by digitising a number of discrete points of the waveform as is for example known from signal theory. The function can, preferably for T-wave, be a wavelet function, one or more spline functions which can be connected or a polynomial function or another function or relation between time and a T-wave signal that a person skilled in signal processing or mathematics would consider to use and that is preferably based on the measured points of the waveform of T-wave. In this embodiment, the apparatus is configured to approximate T-wave information with a mathematical function. The ST-segment can for example be approximated by a linear function, preferably by a segment of a linear function of which more preferably also the end points match with the endpoints of ST-segment. T-wave deviation values or ST-segment deviation values generated by this parameter can be provided and used for a combined grade of activity of a lactate metabolism and activity of a glucose metabolism of the myocardium. As glucose metabolism is an anaerobic metabolism and lactate metabolism is a partially anaerobic metabolism. The parameter can represent anaerobic metabolism of the myocardium. The precision of the parameter is high, because preferably, many data of T-wave are involved in the calculation such that signal noise has a smaller effect. It is also possible to calculate a time period and an amplitude from the function such that also time period data and/or amplitude data can be obtained as a function parameter. They can be used in the same way as time period and/or amplitude parameters that have been acquired in another way. If it is possible to separate an overall amplitude value from an overall time period value in the approximating function in data describing it, equivalents to the parameters amplitude and time period can be calculated by means of approximating the waveform of for T-wave or ST-segment or both by a function.

Further, it is possible to compare parts of measured T-wave information from different T-waves with each other. As a recovery of the myocardium which is represented by T-wave is a process that is not simultaneous throughout the whole myocardium, deviations between certain parts of T-wave can point to a different metabolism in a part of the myocardium that corresponds to a deviating part of T-wave. The correspondence between parts of the myocardium and parts of T-wave is known in cardiology. This can be used for all parameter types. Particularly, anaerobic metabolism, L-metabolism and/or G-metabolism can be distinguished by deviations in area, time and amplitude, respectively. It is also possible to compare few neighbouring or single measurement points of T-wave. Similar considerations can be made for ST-segment

In a further embodiment, the data processing apparatus can be configured to extract assessment data from T-wave information and/or ST-segment information exclusively. In this embodiment, information from other parts of a PQRST-complex are not used for generating assessment data.

In a further embodiment, the data processing apparatus is configured to calculate a normalised T-wave deviation value or ST-segment deviation value by relating the T-Wave deviation value or ST-segment deviation value, respectively, which is based on parameters of T-waves or ST-segments, respectively, to a T-wave characteristic value or a ST-segment characteristic value, respectively of the same parameter which is preferably taken from the same source of T-waves or ST-segment, respectively..

The term "source" means in this context that T-wave information or ST-segment information is taken from a certain place of measurement on same individual, such as from a certain electrode on a certain place on an individual. The absolute amount of deviations which are the main information carrier for the myocardial metabolism usually changes between different sources of a single individual and especially between different individuals though the contained information is the same. The method of this embodiment has the advantage to render the information of the T-wave deviation values or ST-segment deviation values comparable between the sources from one individual as well as between different individuals. This makes it possible to establish standard thresholds for diagnosis of conditions and diseases of the myocardium which can be valid for all or many individual humans or animals of a certain species.

The term "relating" can for example mean a mathematical division or a comparable method known from mathematics or statistics. Also a lookup table to associate the normalised T-wave deviation value to the T-wave deviation value and the T-wave characteristic value can be used. The latter method renders the relation process better adaptable in case that the relationship of the normalised T-wave deviation value to the T-wave deviation value is not proportional. Calculating a division of the T-wave deviation value by the T-wave characteristic value with the result of a normalised T-wave deviation value is a simple and effective method which delivers a good correlation between T-wave characteristics and the myocardial condition as the above mentioned studies of Michael Alekseyev, Aleksandr Alekseyev, Andrew Dowzhikov and Sergei Labin have shown. The methods of relation can also be used for ST-segment deviation values. It is also possible to use T-wave characteristic values for relating of ST-segment deviation values and the reverse. An adapting factor can be used to adapt the values of ST-segment to values of T-wave and the reverse.

A normalised T-wave deviation value or a normalised ST-segment deviation value for the parameter of an amplitude of T-wave or a part of it or of ST-segment is the G criterion of the above mentioned-publication of Alekseyev et al. A correlation of 0.87 has been found between glucose level and G criterion. A normalised T-wave deviation value or a normalised ST-segment deviation value for the parameter of a time period of T-wave or a part of it or of ST-segment is the L criterion of the same publication. Also, deviation values of other parameters of T-wave can be normalised in the same way. A correlation of 0.86 has been found between the percentage (degree) of stenosis in definite coronary artery.

The T-waves or ST-segments used for derivation of the T-wave characteristic value do not have to be the same T-waves or ST-segments from which the T-wave deviation value or of the ST-segment deviation value is derived as far as no basic changes in the individual occur. Of course, the same T-waves or ST-segments or a mix of them can be used in a preferred embodiment.

A "characteristic value" can be a mean value which may be calculated by methods known in mathematics or statistics. It also can mean another value which is typical for the parameter in the examined individual, for example a parameter value that is measured frequently or is a central value of a range of parameter values which are measured frequently. It is also possible to use this principle of relating a deviation value to a characteristic value for other applications as is discussed below. This relates to a characteristic value of T-wave and/or ST-segment.

It is proposed to consider the myocardium as a regulated part of the body and to assess a state of a regulated process of it. Regulated means that the myocardium has a feedback loop in order to stabilise certain body process characteristics. Such a regulation usually does not lead to a constant process but produces deviations in dependency of the regulation mechanism. One such myocardium process can be a metabolism of a myocardium.

A data processing apparatus for detecting a regulation state of the myocardium process is proposed which can measure a certain characteristic of the myocardium process more than one time and to derive a deviation value from at least two measurements of a signal of the body function. The deviation value can relate to a certain parameter of a measured signal from the process, for example an amplitude and/or a time period in the characteristic of the myocardium process. Additionally or alternatively, a deviation of the product of amplitude with time, the integral of amplitude over time, the sum of amplitude values measured at different times, a parameter describing a form of the signal or a part of it and/or a deviation value of an area bordered by the signal or of a function that approximates the signal can be derived. The deviation can be measured and processed to obtain a deviation value. As a myocardium needs stability in most of the processes he carries out, by a deviation value a state of the process of the myocardium can be assessed. A great deviation value means that the regulation process causes great fluctuations of the characteristic which in many cases means a pathological condition of the myocardium.

Information about the myocardium process can be gained by obtaining measurement data from a measurable characteristic which is associated with the myocardium process and which is present in a corresponding signal. To this end, the data processing apparatus comprises a measurement device with at least one measurement path for the flow of information from the characteristic. For example, the measurement path can comprise a measurement data production unit such as an analog-to-digital-converter for generation of amplitude data and/or time data. If more than one measurement path is used, the measurement paths can be switched to the common measurement data production unit. Also, it is possible that two or more measurement paths have its own measurement data production unit. From the measurement data, an amplitude and/or a period of time or a point in time of the characteristic of the body process can be derived. Also, a combination value of amplitude and time, such as an integral of amplitude over time, a sum of amplitude values measured at different times, a form value of a digital signal or/and a bordered area value of the digital signal, wherein the bordered area value comprises information on the an area which is bordered by the digital signal, can be derived from the measurement data. The digital signal comprises the measurement data as points having a time and an amplitude of the signal. The form value is dependent from the form of a segment of the digital signal and a bordered area value is dependent from an area which is bordered by the digital signal an which can further be bordered by an additional border such as an isoline or a line with an offset to zero. The measurement data are preferably measured at known times. Subsequent measurement times preferably have substantially constant time intervals between each other.

Preferably, deviations are measured during a short term measurement period in which raw information is acquired. A short term measurement period means that the deviations are measured in a time period of less than one day and preferably less than 15 minutes, especially during at least 1 second, preferably at least 2, 3 or 4 seconds, more preferably at least 30 seconds and most preferably about 200 seconds. In order to derive second order time deviations, a second short term measurement can be carried out at a later point in time. Between the point in time of the first short term measurement and the second short term measurement, a long-term measurement period elapses. Preferably, the long-term measurement period is longer than the short term measurement period. More preferably, the long-term measurement period is longer than 3 hours which can be applied for acute cases or at least one day for less acute cases or at least one week for non-acute cases but for higher precision of event prediction. The long-term measurement period is used to have a time distance in order to derive a second order time deviation value. Preferably, a high relative resolution of for example at least $5 \cdot 10^{-4}$ is used for the measurement wherein the measurement range is defined as values between a minimum a maximum value of the signal or a relevant part of it.

The deviation value can for example be a difference, especially between two parameters that have been measured one after the other in time or can be a standard deviation of measured parameters or characteristics of the signal or another value derived from a difference between recurring signals of segments of a signal according to a method that is known in mathematics or statistics.

Preferably, a normalisation of the variation is carried out. The purpose of this is to adapt the amount of variation to the myocardium process from which the measurement data are measured such that it can be compared with values of other individuals. In many cases, the measured amount of deviation, the deviation value, is derived from an absolute amplitude, absolute time period values, an absolute product of amplitude and time, an absolute integral of amplitude over time, an absolute sum of amplitude values measured at different times, an absolute form value or function parameter or/and an absolute bordered area value of the measurement data. The assessment data and/or a diagnosis can be normalised by relating the deviation value to a characteristic value as has been discussed in regard of the myocardial condition. Normalised deviation values which are calculated in this way can originate from different measurements at different places or times from the same person or from different persons, wherein the measurements carry information from the same body process. Such normalised deviation values are comparable between different locations of measurement at one person and between measurements at different times. Further, a normalised deviation value from a body can be compared with a normalised deviation value from another body which can have different absolute values. Calculations are preferably carried out by digital data processing.

A data processing apparatus for providing data for assessment of a status of a myocardium process of a body of a human or an animal or a part of it is proposed as an optional embodiment. The apparatus comprises a measurement device which is arranged to measure a characteristic of a process of the myocardium with at least one measurement path for information which is retrieved from the myocardium, and to produce measurement data of the at least one

7

measurement path. Further, the apparatus comprises a calculation device which is arranged to calculate a deviation value from the at least one measurement path. The apparatus can further comprise a normalisation device that provides a normalised deviation value for each measurement path which each corresponds to the deviation value of the measurement path related to a characteristic value of the measurement data. The measurement data used for calculation of the characteristic value are preferably from the same measurement path.. The characteristic value then belongs to this measurement channel. It also can be a characteristic value that is calculated from measurement data that originate from different measurement paths. Then, the characteristic value is a more general characteristic value which can for example be used for relating it to an average value of measurement data of some or preferably all of the different measurement paths used for calculation of the more general characteristic value. Such an average value is calculated from measurement data acquired in a shorter period of time than the measurement data for calculation of the characteristic value. Preferably, the average value is calculated from measurement values that have been acquired from one single acquisition from each of the relevant different measurement paths. By relating an average value to a more general characteristic value of, a normalised average value is calculated. Further, the apparatus can comprise a comparison device which is configured to compare a deviation value or a normalised deviation value or an average value or an normalised average value to a threshold value. Such a threshold can for example be obtained by experiments in which common methods for diagnosis for heart conditions or diseases are compared with the corresponding values according to the diagnosis methods based on the data processing apparatus or a corresponding data processing method as disclosed in this patent application. Additionally or alternatively, the comparison device can be configured to compare deviation values or normalised deviation values or average values or normalised average values of the same type, but measured at different times or different locations, to each other. Particularly, this can be useful to track a condition or a disease of a person. Additionally or alternatively, the comparison device can be configured to calculate a quotient of two deviation values or normalised deviation values from different measurement paths. The quotient can be compared to a threshold. Single or groups of devices of the apparatus can be arranged in different units which preferably can be locally separated from each other. One or more of the processes carried out by the devices of the apparatus can form a method for providing concerning myocardium or for assessing the condition of the myocardium.

In a further embodiment, the apparatus is configured to compare a T-wave deviation value or a normalised T-wave deviation value of a first source of an individual with a T-wave deviation value or a normalised T-wave deviation value, respectively, of a second source of the same individual and to generate a local deviation value. A source can be an electrode which is placed on the body of the person or the animal to preferably measure a certain part of the heart or a certain part more than other parts. It is also thinkable that a source can be an electric or electromagnetic field from the heart which are preferably received from a certain part of the heart by a suitable receiver. The first and the second source yield information from different locations at the heart. For example, second order local deviation values can be obtained by calculation of differences between information from different locations. Deviation values on which this calculation is based are derived from parameters of T-wave in a first step are called first order deviation values in this context. They are preferably normalised deviation values as described above. So, second order deviation values can be obtained from deviations between first order deviation values regarding location as described above or regarding time as described below. In order to obtain one second order local deviation values, four measurements are required. At least two of these measurements are carried out using the first source and at least two measurements are carried out using the second source.

As mainly the leads V2, V3, V4, V5 and V6 belong to the left ventricle which is affected most often from ischemic heart disease, these leads are preferably involved in generating a local deviation value. A comparison between different leads is advantageous, because local differences can be recognised which are a hint to an ischemia. The myocardium can have a locally deviating metabolism which can go along with a probability of an acute myocardial infarction. The locations of the electrodes can be the standard locations.

There might be cases in which normalisation is not necessary for comparison between different sources of an individual, for example in individuals which have very similar T-waves from different sources. However, this is not the normal case, but still, it is possible. For example, the leads V1, V2, V3, V4, V5 and V6 can be compared one with another to generate subtraction values or to generate relation values, for example values that represent a quotient.

In general, it is not required to use leads V1 to V6 according to Wilson. It is also possible, with less local resolution, to use the leads aVR, aVL and aVF according to Goldberger. It is also possible to use further known arrangements of electrodes and leads.

In case of using the unipolar leads according to Goldberger, it is preferred to carry out measurements between different combinations of the leads. Then, quotients or differences of two of the results an be calculated, respectively. A comparison between the quotients or differences can be interpreted as a second order local deviation value. With a second order local deviation value can be assessed whether there are local differences of a disease in the myocardium. This can also be done with the standard Einthoven electrode and lead arrangement with leads I, II and III.

T-wave deviation values can be compared with ST-segment deviation values in order to generate a second order deviation value. Normalised T-wave deviation values can be compared with normalied ST-segment deviation values in order to generate a normalised second order deviation value.

In a further embodiment, the data processing apparatus is configured to process a number of at least 3 T-waves, preferably at least 60 T-waves and most preferably about 200 T-waves and/or to process a number of T-waves of a PQRST-complex during at least 1 second, preferably at least 2, 3 or 4 seconds, more preferably at least 30 seconds and most preferably about 200 seconds to obtain normalised T-wave deviation values or T-wave deviation values and to calculate a second order deviation value of the obtained normalised T-wave deviation values or T-wave deviation values. As at least three measurements are necessary to carry out a second order deviation, at least three heartbeats are acquired which can be acquired in one second at very fast heart rate and in 2, 3 or 4 seconds with slower heart rates. With a number of at least measured 60 T-waves, the quality of the data produced by the data processing apparatus can be improved significantly by better statistics. A good trade-off between measurement time and high-quality is acquiring about 200 T-waves which corresponds to measurement time of typically 200 seconds with a sitting patient which has quite slow heart rate. For even better quality, longer measurements times can be used. The first order deviation information is contained in the T-wave deviation values or normalised T-wave deviation values, wherein the second order deviation information is contained in differences between the normalised T-wave deviation values or T-wave deviation values from different sources or measured at different times.

In a further embodiment, the data processing apparatus can be configured to compare a T-wave deviation value or a normalised T-wave deviation measured at a first time to a T-wave deviation value or a normalised T-wave deviation value, respectively, measured at a second time, wherein the second time is different from the first time, and to generate a time deviation value. As this results in a second order deviation regarding time, at least three measurements of T-wave are required. A first difference between a first combination of two of the T-waves parameters delivers a first first order deviation value and a second difference between a second combination of the T-waves delivers a second to first order deviation value. The first and the second to first order deviation value can be compared to obtain their second order time deviation value. Preferably, the values are obtained as normalised deviation value, wherein the normalised deviation values are calculated from deviation values by division by a characteristic value as described above. It is also possible to divide a second order time deviation value which is based on non-normalised first order deviation values by a characteristic value. By such a second order time deviation value, a trend of the T-wave deviation value or the normalised T-wave deviation value can be represented. Non-normalised values can for example be used for one single individual. When a comparison between individuals is required, the normalised values are preferred. With such a trend, an event time period or an event point in time can be extrapolated after which or at which, respectively, it is expected that the T-wave deviation value or the normalised T-wave deviation value reaches a predefined value, for example a threshold value. Extrapolation methods are well known in mathematics, information technology and engineering. In this way, a prognosis can be made when a certain condition of the heart can be expected. For example, an event time period can be calculated after which an acute myocardial infarction, a sudden heart death, death by syndrome X, a certain degree of stenosis, a certain degree of angina pectoris and/or another NYHA-class according to the New York Health association (NYHA) can be expected. The conditions mentioned above can be derived from T-wave information as described in this patent application.

The proposed measurement of a second order deviation value preferably takes at least 1 to four seconds, preferably at least 30 seconds f overall measurement time which provides better data quality and more preferred about 2 to 3 minutes of overall measurement time which provides an optimum trade-off between quality and measurement time. For even better quality, longer measurements times can be used. With such a data processing apparatus, a development of a myocardial metabolism can be represented in a value which is based on one or more parameters.

Particularly, from the change rate of non-normalised or normalised T-wave deviation values or T-wave deviation values, an expected time to an acute myocardial infarction can be calculated. The change rate is represented in the second order time deviation value, such that the second order deviation value can be a basis of such a calculation. The calculation can be based on a lookup table or a function which is based on a typical correlation between the time to an acute myocardial infarction and a normalised T-wave deviation value or T-wave deviation value, preferably the L criterion. There is also a correlation to the G criterion, but it is less strong. The correlation can for example be known from example cases and/or clinic studies and/or calculation of the metabolism, especially based on supply, use and storage level of lactic acid and/or glucose.

A second order distance in time of the deviation values for calculation of a second order time deviation value or a normalised second order time deviation value is preferably greater than a first order distance in time of the measurement values for the first order deviation values on which the non-normalised or normalised second order time deviation values is based.

The different measurements to derive a second order time deviation value preferably originate from the same location but are measured at different times such that a second order local deviation value or a normalised second order local deviation value can be obtained.

In a further embodiment, the data processing apparatus comprises a measurement device for generating T-wave information from an individual human or animal by measurement, wherein the measurement device preferably has a resolution of less than 5ms, or less than 285$\mu$s, preferably less than 100$\mu$s and most preferable less than 50$\mu$s in time and/or of less than 50mV or less than 16$\mu$V in amplitude, preferably less than 1$\mu$V and most preferable less than 1 nV. Preferably, in these values also the precision and/or interference into electrodes and/or leads at standard conditions in a hospital is comprised.

The data processing apparatus has preferably one or more features of a common electrocardiograph with improved resolution. Particularly, it can comprise a common number of lines and electrodes. Though measurement with common electrodes is possible, electrodes for the measurement device are preferably low noise electrodes. Also, wireless electrodes are preferred.

In a further embodiment, the data processing apparatus comprises a signalling means which is configured to signalise and/or display a deviation value and/or a normalised deviation value of a parameter and/or a diagnosis proposal and/or a diagnosis that is made automatically to a person and/or a risk for a heart condition and/or a heart disease. The diagnosis proposal and/or the diagnosis and/or the risk for a heart condition and/or a heart disease is derived from the measurements and/or parameters as explained below. For example, non-normalised or normalised deviation values of amplitude, time period, area and/or function parameters, event time period or event point in time, risk and/or presence and/or extent of: stenosis, myocardial infarction, particularly acute myocardial infarction, syndrome X, sudden heart death, angina pectoris and/or NYHA-class can be signalled as a single information or an arbitrary combination. Preferably, the information can be signalled or displayed for each lead separately. The signalling means can be arranged at another location as the data processing apparatus or are measurement device which is connected to the human or animal. The signalling means and the data processing apparatus can for example communicate via a data link or physical memory can be exchanged.

In a further embodiment, the data processing apparatus comprises a data acquisition means comprising a data memory which is configured to record deviation data or normalised deviation data or diagnosis proposal data or automatic diagnosis data and/or a data remote transfer means to transfer deviation data or normalised deviation data or diagnosis proposal data or automatic diagnosis data to a remote means of the data processing apparatus, wherein, preferably, the data acquisition means is locally separable from a signalling means at which data from the data memory can be displayed.

In a further embodiment, a non-normalised or normalised first order or second order T-wave deviation value represents a variable to assess if and/or to which extent a metabolism of the heart and/or a heart condition and/or heart disease is active. A decision about presence or absence of a metabolism type and/or a heart condition and/or heart disease can be derived from the T-wave deviation value, but also different extents of a metabolism and/or a heart condition and/or heart disease as well as risks for those conditions and/or diseases. The assessment of the extent of presence of a metabolism and/or extent and/or risk of a heart condition and/or heart disease allows for much more precise assessment of the severity of a condition, especially an ischemic heart disease. T-wave deviation values and normalised T-wave deviation values as well as second order deviation values can for example be displayed by the apparatus.

In a further aspect of the invention, an assessment apparatus for an assessment of a condition of at least a part of a myocardium or the whole myocardium of a heart of an individual human or animal and/or a heart disease is proposed. This can also comprise assessing an event time period or an event point in time as described above. The assessment apparatus can use a T-wave deviation value or/and a normalised T-wave deviation value or/and a second order local deviation value or/and a second order time deviation value which have been obtained by a data processing apparatus as described above. A. Data provided by the data processing apparatus are considered assessment data. The assessment apparatus can be integrated into the data processing apparatus. Additionally or alternatively, assessment data can be supplied by data transfer from the data processing apparatus or by reading out a memory which has been recorded by a data processing apparatus.

In an embodiment, the assessment apparatus comprises a data processing apparatus according to one of the embodiments described in the present patent application. This does not necessarily mean that the assessment function is executed in the same location as the data processing, but it can also be the case. Alternatively, the assessment can be done locally separated from the data processing and/or the data acquisition from the human or animal. A centralised assessment can be used for assessment of assessment data from a plurality of data processing apparatuses. It is also possible to have the data processing apparatus and the assessment apparatus at the same location and to process and assess acquired data from a plurality of humans or animals which have been acquired at another location as the lovcation o the data processing apparatus and the assessment apparatus.

In an embodiment of the assessment apparatus, the assessment apparatus is configured to assess

A) risk and/or presence and/or extent of ischemic heart disease by a T-wave deviation value (1DV) or a normalised T-wave deviation value (N1DV) or a ST-segment deviation value or a normalised ST-segment deviation value of parameter time period, and/or by a T-wave deviation value (1 DV) or a normalised T-wave deviation value (N1DV) or a ST-segment deviation value or a normalised ST-segment deviation value of parameter amplitude, and/or

B) risk and/or presence and/or extent of stenosis by a T-wave deviation value (1DV) or a normalised T-wave deviation value (N1DV) and/or a ST-segment deviation value or a normalised ST-segment deviation value of parameter time period, and/or

C) risk and/or presence and/or extent of angina pectoris caused by a heart condition or disease by a T-wave deviation value (1 DV) or a normalised T-wave deviation value (N1 DV) or a ST-segment deviation value or a normalised ST-segment deviation value of parameter time period and a T-wave deviation value (1 DV) or a normalised T-wave deviation value (N1DV) or a ST-segment deviation value or a normalised ST-segment deviation value of parameter amplitude or an area parameter or one or more function parameters, and/or

D) risk and/or presence and/or extent of syndrome X by a T-wave deviation value (1 DV) or a normalised T-wave deviation value (N1DV) or a ST-segment deviation value or a normalised ST-segment deviation value of parameter time period and a deviation value of parameter amplitude or an area parameter or one or more function parameters, and/or

E) risk and/or presence and/or extent of myocardial infarction, especially acute myocardial infarction, by a T-wave deviation value (1DV) or a normalised T-wave deviation value (N1DV) or a ST-segment deviation value or a normalised ST-segment deviation value of parameter time period, and/or by a quotient of a a T-wave deviation value (1 DV) or a normalised T-wave deviation value (N1 DV) or a ST-segment deviation value or a normalised ST-segment deviation value of parameter time period and a T-wave deviation value (1 DV) or a normalised T-wave deviation value (N1 DV) or a ST-segment deviation value or a normalised ST-segment deviation value of parameter amplitude, and/or

F) risk and/or presence and/or extent of sudden heart death by a T-wave deviation value (1 DV) or a normalised T-wave deviation value (N1 DV) or a ST-segment deviation value or a normalised ST-segment deviation value of parameter amplitude, and/or

G) a NYHA classification by a T-wave deviation value (1 DV) or a normalised T-wave deviation value (N1 DV) or a ST-segment deviation value or a normalised ST-segment deviation value with parameter amplitude, and/or

H) an event time period or an event point in time for a condition or a disease of the heart as mentioned in item A) to G) by a second order time deviation value (2TDV) or a normalised second order time deviation value (N2TDV) of a parameter as mentioned under A) to G), in respect of a certain condition or disease, particularly an event time period to an acute myocardial infarction or an event point in time of an acute myocardial infarction by a second order time deviation value (2TDV) or a normalised second order time deviation value (N2TDV) of parameter time period or by a second order time deviation based on a quotient of parameters time period and parameter amplitude, and/or

I) a location at the heart of a condition or a disease of the heart as mentioned as item A) to G) by a second order local deviation value (2LDV) or a second order local deviation value (N2DLV) of a parameter mentioned under A) to G) for the condition or disease, respectively.

As the assessment of the condition relates a continuous value which which can be assessment information and which by itself does not have any thresholds to a condition or disease, it is possible to assess the extent of a condition or disease. Therefore, also conditions can be recognised which not yet have expressed themselves as a disease. In this way, a risk for the disease or a worse condition can be assessed. In contrast, a diagnose is made by comparison of assessment information with a threshold. An assessment can comprise providing assessment data in a context of a certain condition which is correlated to the assessment information. An assessment can also comprise finding one or more conditions or diseases on basis of a single assessment information which can be a first order or second order deviation value, or a combination of such assessment information. The assessment of a disease or condition can additionally be made dependent on the situation or condition in which a patient is.

For example, if a patient comes to a doctor or a hospital with chest pain, it can be distinguished whether the chest pain is caused by an angina pectoris or by another cause which is located in the chest. It is also possible to find out about the presence of syndrome X without doing an angiography. Syndrome X will not be detected if a patient does not have an angina pectoris, by definition of syndrome X. However, by using a method as described in the present patent application, it can be detected that a stenosis of the coronary arteries is not present.

In another example, a person comes for a routine control without angina pectoris. Then, the presence of an ischemic heart disease can be detected which also can be present without angina pectoris. Ischemic heart disease comprises a few different and more special diseases like stenosis, myocardial infarction, NYHA-classification which is beyond normal or sudden heart death. If an increased deviation value or normalised deviation value of parameter amplitude can be detected, there is an increased risk for a sudden heart death. Particularly, if the patient is a sportsman or should do heavy exercise like a stress electrocardiogram, sudden heart death can occur. Advantageously, the patient can be warned or the stress electrocardiogram can be omitted. A NYHA-classification can for example be conducted on basis of a deviation value or normalised deviation value of parameter amplitude, instead.

In a further example, a patient whose myocardium has been measured according to the invention comes to the next routine control. By differences between the information taken from his myocardium the first time and the second time, the development of the condition of the myocardium can be assessed. It can also be interpolated into the future, for example for early warning regarding a myocardial infarction or another NYHA-class.

In a further example, only a part of the myocardium is affected by a disease, for example by a stenosis. Then, it is possible that this disease extends to further parts of the myocardium. For example, an infarction of only a part of the myocardium can lead to dysfunction of the whole heart. Therefore, such differences between different leads by which can be recognised that only a part of the myocardium is concerned can be taken into account, as an average value over all leads can be insignificant.

In a further embodiment, it is proposed to calculate a difference or a quotient between two deviation values or normalised deviation values of two different leads. In this way, the differences between the values of two different leads can be expressed in one single second order local deviation value. It is also possible to multiply the difference between two deviation values or normalised deviation values of two different leads by a mean value of the values on which the difference value is based. As mentioned above, also differences in the results which are measured using the Goldberger lead arrangement can be interpreted in this way. Whereas using a quotient is comparable between different individuals, a multiplication is less comparable but changes in a stronger way with the differences between the leads.

The results of data processing, for example deviation values of first and second order, can be considered as being symptoms. On basis of these symptoms, an assessment of a condition of the myocardium or a diagnosis can be carried out. This can be done by a person that can interpret the assessment data or it can be done automatically to generate a diagnosis proposal and/or a diagnosis and/or diagnosis proposal data and/or diagnosis data. In comparison, an assessment is considered an extent of a disorder whereas a disease is diagnosed as a disorder above a certain level. The diagnosis is made by comparison of a symptom with a threshold value which can be carried out by an assessment apparatus with this additional function as disclosed below. Preferably, the assessment apparatus comprises a diagnosis device for generating a diagnosis proposal or an automatic diagnosis.

In a further embodiment, the assessment apparatus is configured to carry out the assessment in form of an automatic diagnosis by a comparison of a criterion to a threshold value in order to come to a decision about a condition and to preferably provide a diagnosis proposal according to the assessment as described in this application and/or a diagnosis which is made automatically according to the assessment as described in this application and/or an automatic alarm for a user of the apparatus and/or a medical service when a decision about a high risk of an acute myocardial infarction has been made.

For example, a threshold value of 11 for the normalised T-wave deviation value based on the parameter time period (L-criterion) can be set for an assessment that an acute myocardial infarction will occur in few hours. The L-criterion is dimensionless. The time to a myocardial infarction can be calculated by the time that is needed for the value to reach the threshold value, by interpolation.

An ischemic heart disease can for example be recognised by a normalised deviation value of parameter time period of 1 or greater, preferably 1.5 or greater and/or by a normalised deviation value of parameter amplitude of 1 or greater, preferably greater than 1.5. A stenosis can be detected by a normalised deviation value of 2 or greater, preferably 2.5 or greater.

Angina pectoris can be distinguished from another type of chest pain by a normalised deviation value of parameter amplitude of 1.5 or greater and a normalised deviation value of parameter time deviation which is 1.5 or greater. A risk of syndrome X can be detected by a normalised deviation value of parameter time period of 1 or greater, preferably of 1.5 or greater and by a normalised deviation value of parameter amplitude of 1 or greater, preferably of 1.5 or greater. A NYHA class which means the presence of a heart disease can be recognised in the same way an ischemic heart disease can be detected. Thresholds for the NYHA classes I to IV can be determined by a person skilled in the art in correlating normalised deviation values of parameter amplitude and/or parameter time period which have been measured from a patient to the NYHA class of this patient. This can be done with many patients to have a good statistic.

In a further aspect of the invention, a method for processing T-wave information of an electrocardiogram for an assessment of a condition of at least a part of a myocardium of a heart of an individual human or animal is proposed. The method can use the features, method steps and processes that are defined in regard of the apparatuses which are described in this patent application.

Further, a method for assessing a condition of at least a part of a myocardium of a heart is proposed. In this method, assessment data which have been generated by an apparatus or a method of one of the aforementioned embodiments is compared to a threshold value to obtain condition information. The comparison is preferably done automatically. A diagnosis proposal according to the assessment as described in this application and/or a diagnosis which is made automatically according to the assessment as described in this application and/or an automatic alarm for a user of the apparatus and/or a medical service when a decision about a high risk of an acute myocardial infarction has been made can be provided.

In a further aspect of the invention diagnosis method is provided, according to which the diagnosis of a condition or a disease of a heart is made by applying an assessment as described above.

Embodiments of the invention, as an example only, are depicted in the attached figures, in which

Figure 1    shows a schematic diagram of a measurement of T-wave information from a human heart,

Figure 2    schematically shows a PQRST complex which can be measured from a human heart,

Figure 3    schematically shows a T-wave and parameters which can be used to derive a deviation value,

Figure 4    shows a schematic diagram of flow of information in a data processing apparatus,

Figure 5    shows a schematic diagram of flow of information in an assessment apparatus,

Figure 6    shows a schematic diagram of relationships between a parameter of a T-wave and conditions of a myocardium,

Figure 7    shows a schematic diagram of flow of information from the myocardium to normalised T-wave deviation values,

Figure 8    shows a schematic diagram of flow of information from normalised T-wave deviation values of the myocardium to a second order deviation value, and

Figure 9    shows a schematic diagram of flow of information from normalised T-wave deviation values the myocardium to a second order time deviation value.

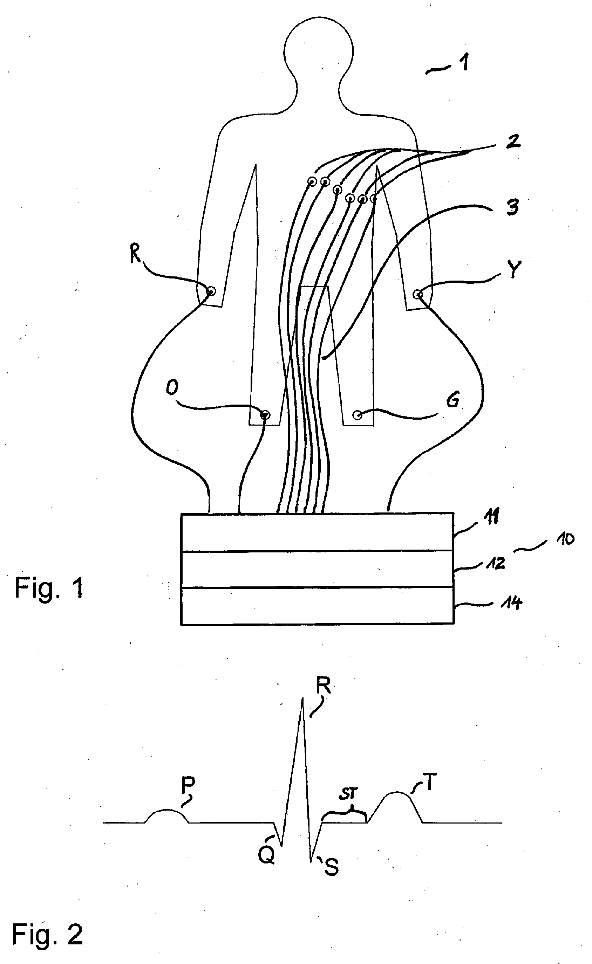

Figure 1 schematically shows a human 1 in a face-to-face front view. On his upper part of the body, six electrodes 2 are applied for measurement of electric signals produced by the heart of the human 1. The electrodes 2 are connected to a measurement device 11 via cables 3 which together form the well-known leads 1 to V6. Alternatively, wireless electrodes can be used. The measurement device 11 produces T-wave information from T-waves in the electric signals. The measurement device 11 is connected to a data processing apparatus 12 and forwards the T-wave information to it. The data processing apparatus 12 derives deviation values from the T-wave information. The deviation values can be normalised or non-normalised deviation values and considered as assessment data. The assessment data are input into an assessment apparatus 14. The assessment apparatus 14 assigns the condition or a disease of a myocardium or part of it or the heart of the human 1 on basis of the assessment in data. The condition or disease can be displayed, transferred to an external unit for further use for example for external recording or alarming a medical staff or service and/or saved in a memory.

In Figure 1, the reference leads R at the right hand, G at the left hand, Y at the left foot, and 0 at the right foot are shown. As is known in cardiology, a virtual reference point for the leads V1 to V6 from the electrodes 2 can be calculated form signals that are present between the leads R, Y and G with reference to lead 0. It is preferred for more precise results to do so in the present invention, also. The calculation of the reference signals in the torso can be executed as follows:

$$QVR = R/(Y*G), \quad QVL = Y/(R*G) \quad \text{and} \quad QVF = G/(R*Y)$$

These virtual reference points are located in the torso and thus closer to the heart. It is possible to use a mean value of QVR, QVL and QVF as a reference, also. It is also possible to exchange the leads 0 and G such that G is the reference for the leads R, Y and 0.

Figure 2 schematically shows a PQRST compex which can be measured electrically from a human heart. The waveform represents a voltage which is plotted in vertical direction over time which is displayed in horizontal direction. A T-wave T appears after the QRS complex. A ST-segment is labelled with the reference sign ST.

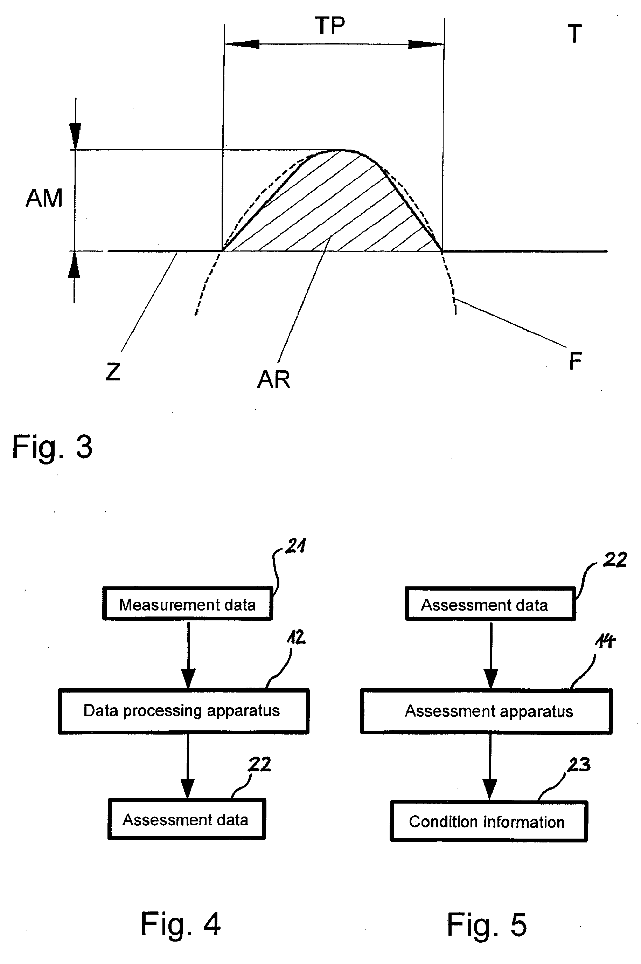

Figure 3 schematically shows a T-wave T as a voltage waveform plotted over time and parameters TP, AM, AR and F which can be extracted from the T-wave information which is contained in the waveform of T-wave.

The time period parameter TP is a parameter regarding time that corresponds to the length in time of T-wave T. T-wave T begins in case of the shown type of T-wave T when T-wave T starts rising above the isoline Z and ends when T-wave T reaches the level of the isoline Z again. In other types of T-wave T, the start and end can be at other voltage levels, for example when the ST-line has another level as normal, for example a higher level. It is also possible that the T-wave T extends to negative voltage from the isoline Z. Then, the T-wave T starts when it is falling below the isoline Z and ends when T-wave T reaches the level of the isoline Z again.

Further, the amplitude parameter AM can be extracted from T-wave information. It is the difference between a maximum of T-wave T and a point of beginning or end of T-wave. Also, it can be a difference between a maximum of T-wave T and the isoline Z.

Further, area parameter AR can be extracted from T-wave information. Parameter AR is derived from an area AR which is partially, in many cases at its upper rim, bordered by T-wave T. The area AR can for example further be bordered by a prolonged isoline, as is shown in Figure 3. As only deviations of the parameters are relevant, it is not important where the further border of the area AR is as long as this border is constant, because its information is eliminated when a subtraction is carried out in order to derived a deviation between the parameters AR of two T-waves.

Further, an approximation parameter F can be extracted from T-wave information. An approximation function is shown in Figure 3 as a dotted line. It approximates the waveform of T-wave T, preferably as a section of the approximation function. The approximation function can be described by approximation parameter F which can comprise more than one information such as coefficients of a polynomial function. for example a spline function, or a wavelet or a Fourier transformed function of T-wave or of another function. Preferably, measured points lie in the function. They can form nodes of the function. Differences between parameter F of different T-waves can be used to derive deviation values.

Figure 4 schematically shows a data flow through a data processing apparatus 12.

Measurement data 21 which originate from a measurement of T-wave are input into the data processing apparatus 12. The measurement data can also be measurement signals. The data processing apparatus 12 derives first and/or second order deviation values which can be normalised or non-normalised, from the measurement data 21. The deviation data can further be processed to obtain assessment data 22 or to serve as assessment data as such. For example, a skilled person can assess a condition or diagnose a disease by first and/or second order deviation values. Fro example, current deviation vaues can be compared with devation values which have been measured before. Assessment data 22 are output from the data-processing apparatus 12.

Figure 5 schematically shows a data flow or through an assessment apparatus 14. Assessment data 22 from a data processing apparatus 12 are input into the assessment apparatus 14. The assessment apparatus 14 can compare assessment data with a threshold in order to assess a condition of a myocardium. Corresponding condition information 23 can be output from the assessment apparatus 14 by a display or another signal and/or the condition information can be recorded and/or transferred to an external unit. The condition information can for example be a diagnosis proposal or and automatically made a diagnosis. Such a diagnosis proposal or diagnosis can comprise a condition or disease type, a risk for a condition or disease type, and extent of the present condition or disease, a time period after which a certain event regarding the the condition or disease is expected or a point in time at which this event is expected. Such an event can for example be the transition into another NYHA-class or an acute myocardial infarction or a sudden heart death or an end of acute angina pectoris.

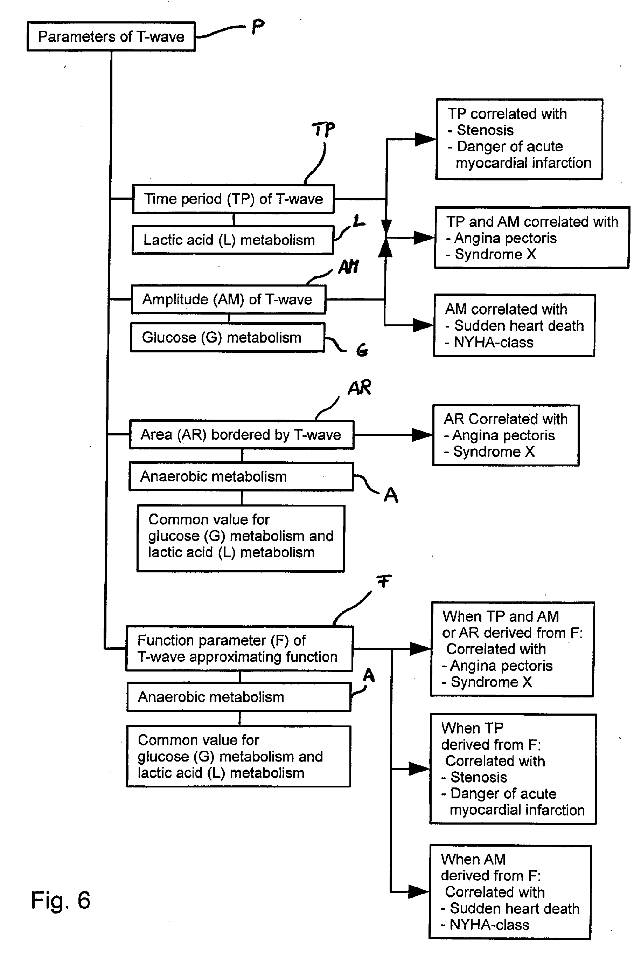

Figure 6 schematically shows a diagram of different parameters P of T-wave and their correlations to different conditions and diseases of a myocardium or a heart.

Time period parameter TP is correlated to activity of a lactic acid metabolism L. The presence of a lactic acid metabolism L, starting from a certain amount which is normal for a myocardium, is correlated to a stenosis and the danger of acute myocardial infarction.

Amplitude parameter AM is correlated to activity of a glucose metabolism G. The presence of a glucose metabolism G is correlated with sudden heart death and the New York Health Association (NYHA) class as a condition of a heart.

Area parameter AR and function parameter F each are correlated with glucose metabolism G and lactic acid metabolism L. If information of both metabolisms G and L are intermingled, this also represents a value for a generally anaerobic metabolism A. Anaerobic metabolism A is correlated to angina pectoris and syndrome X. The presence of anaerobic metabolism can b e derived from parameters AR and F as well as from a combination of parameters AM and TP.

Risk, presence and extent of a stenosis can be assessed by the parameter time period TP of T-wave. By the same parameter, risk, presence and extent of an acute myocardial infarction can be assessed.

Risk, presence and extent of a sudden heart death event can be assessed by the parameter amplitude AM. By the same parameter, a NYHA-class can be assessed. Risk, presence and extent of syndrome X can be assessed by a combination of the parameters time period TP and amplitude AM, preferably a multiplication, or an integration of amplitude AM along the time period TP can be used as a way of combination. Also, an area AR which is partially bordered by T-wave correlates with syndrome X. A corresponding value can also be derived from one or more function parameters F which approximate T-wave by a function.

From the function parameters F, also the time period TP and/or the amplitude AM can be derived. The time period TP and amplitude can then be used for the assessments described above.

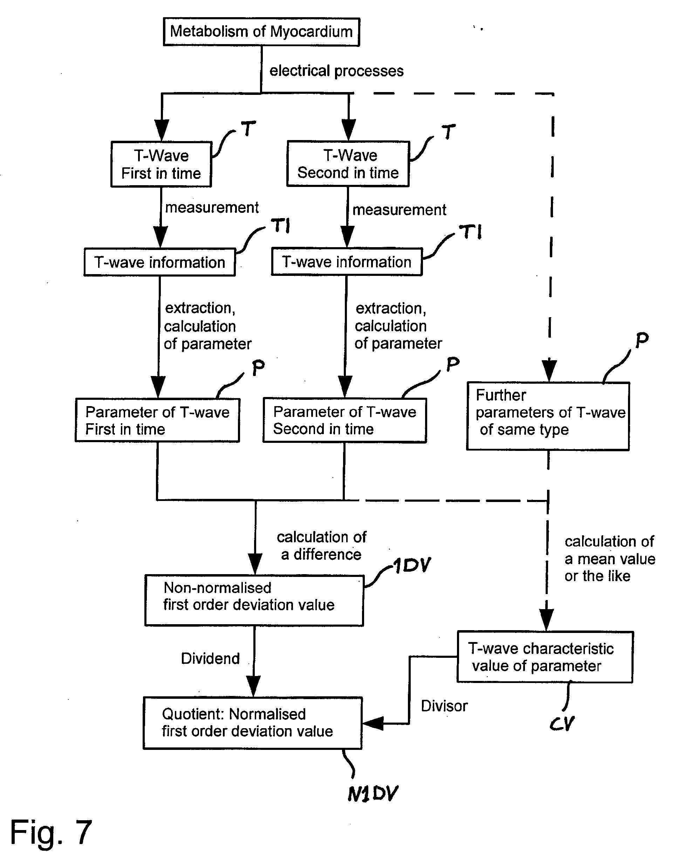

Figure 7 schematically shows a diagram of information flow from the metabolism of a myocardium which produce electrical signals to a normalised T-wave deviation value of first order. At the beginning of the information chain is the metabolism of the myocardium that comprises electrical processes which are present when the myocardium recovers and which can be measured as a T-wave. Following each other in time, several T-waves T are measured, two of which are shown in Figure 7. The measurement of the T-waves T produces T-wave information TI for each T-wave T. Then, from each T-wave information a parameter P is extracted or calculated. The parameter types are the same. In the next step, a deviation 1 DV regarding the parameters P is derived. As this is the first setting into relation of the two parameters P, the result this is called first order deviation value 1 DV, which is non-normalised at this stage.

In order to calculate normalised T-wave deviation values N1 DV from the T-wave deviation values DV, the T-wave deviation values are divided by a T-wave characteristic value CV which is derived from the same type of parameter P as the T-wave deviation values DV. In Figure 7, further parameters P of T-wave of he same type as the other parameters P is shown. Preferably, the T-wave characteristic value CV is a mean value of parameter values of T-wave of which the first order deviation values 1DV have been derived. The normalised T-wave deviation values N1 DV can be used as an assessment data. It is also possible to renounce of the normalisation. Then, the T-wave deviation values 1 DV can be used as assessment in data. This is for example possible, if data are used from one single individual only. For example, a change during measurement time of a T-wave deviation value based on a time period parameter measured from one source of a human does not necessarily need normalisation in order to assess a risk of an acute myocardial infarction, for example. However, it is preferred to have a universal system with normalisation which can used on every individual of a species.

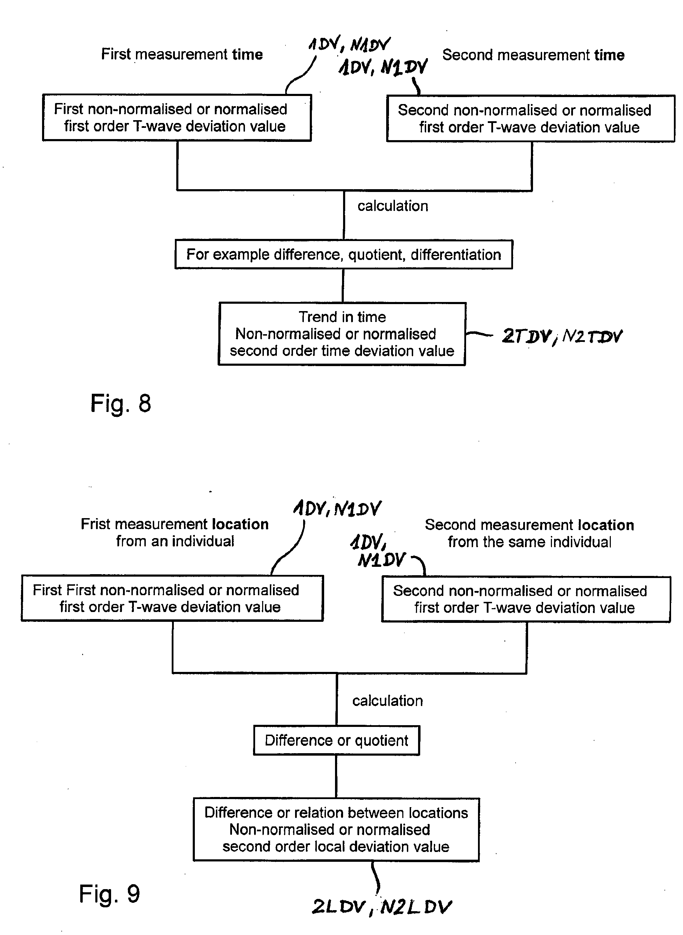

Figure 8 schematically shows an information flow from normalised T-wave deviation values 1DV, N1 DV to a second order time deviation value 2TDV, N2TDV. In order to find a trend of a first order T-wave deviation value 1DV or normalised T-wave deviation value 1 NDV, T-wave deviation values 1DV or normalised T-wave deviation values 1 NDV which have been taken at different times can be compared, especially subtracted from each other or divided one by the other, to derive a second order time deviation value 2TDV, N2TDV. The second order time deviation value 2TDV, N2TDV comprises information of how much the T-wave deviation value 1 DV or normalised T-wave deviation value N1 DV changes in time. When the T-wave deviation value 1DV or the normalised T-wave deviation value N1 DV is based on time period parameter TP, a risk and/or a an event time period and/or an event point of time of acute myocardial infarction can be assessed.

Figure 9 schematically shows an information flow from normalised T-wave deviation values 1 NDV, N1 DV to a local deviation value 2LDV, N2LDV. In order to find a local deviation of a first order T-wave deviation value 1 DV or normalised T-wave deviation value 1 NDV, T-wave deviation values 1DV or normalised T-wave deviation values 1 NDV which have been measured at different locations and which are thus influenced by different parts of the myocardium can be compared, especially subtracted from each other or divided one by the other, to derive a second order local deviation value 2LDV or second order normalised local deviation value N2DLV. The local deviation value LDV comprises information of how much the T-wave deviation value 1 DV or normalised T-wave deviation value N1DV changes in regard of the location of measurement.

In a preferred embodiment, an information flow that is shown in figures 6 to 9 is realised. The parameters P of T-wave T as shown in Figure 6 from which the first order T-wave deviation values 1DV, N1 DV and the second order T-wave deviation values 2TDV, N2TDV, 2LDV, N2LDV are calculated as shown in Figures 7, 8 and 9, are based on the time period parameter TP and on an amplitude parameter AM of T-wave T, such that two types of first order T-wave deviation values 1 DV, N1 DV and second order T-wave deviation values 2TDV, N2TDV, 2LDV, N2LDV are derived. This derivation is carried out by subtraction of starting point time from an end point time of T-wave and subtraction of a minimum amplitude value a from a maximum amplitude value of T-wave T, respectively. A T-wave characteristic value CV is calculated as a mean value of the parameters which have been used as a basis for calculation of a deviation value DV, respectively. Therefore, two T-wave characteristic values CV are calculated, one for the time period parameters and one for the amplitude parameters. The normalised T-wave deviation values N1 DV are calculated by dividing the T-wave deviation values 1 DV by the T-wave characteristic values CV. The same is true for parameters area and function, analogously.

## Claims

1. Data processing apparatus (12) for processing T-wave information (TI) or ST-segment information from a signal from a myocardium of a heart of a human or animal, for an assessment and/or a diagnosis of a condition or a disease of at least a part of the myocardium or the whole myocardium,
   **characterised in that** the data processing apparatus (12) is configured to derive a T-wave deviation value (DV, NDV, 2DV, N2D) from a difference of at least two T-wave parameters (P) or ST-segment parameters,
   wherein the two parameters (P) belong to a T-wave (T) or a ST-segment (ST) from different heart beats of the same heart.

**2.** Data processing apparatus (12) according to claim 1, **characterised in that** the T-wave information (TI) or ST-segment information for obtaining the parameters (P) has a resolution of less than 5 ms, preferably less than 285µs, preferably less than 100µs and most preferably less than 50µs in time and/or of less than 50mV preferably less than 16µV in amplitude, preferably less than 1µV and most preferable less than 1 nV.

**3.** Data processing apparatus (12) according to claim 1 or 2, **characterised in that** the parameter (P) is

> A) a time period (TP) of the T-wave (T) or the ST-segment (ST) or a characteristic part of it and/or
> B) an amplitude (AM) of the T-wave (T) or the ST-segment (ST) or a characteristic part of it and/or
> C) an area (AR) which is partially bordered by the T-wave (T) and/or the ST-segment (ST) and/or
> D) a function parameter (F) of a function approximating or matching the T-wave (T) and/or the ST segment (ST), wherein the data processing apparatus (12) is configured to approximate or describe T-wave information (TI) or ST-segment information with a mathematical function,

wherein, preferably, the parameter (P) is provided for assessment or diagnosis of a heart condition or a heart disease.

**4.** Data processing apparatus (12) according to one of the preceding claims, **characterised in that** two parameters (P) of the same parameter type are set into relation to each other to derive a deviation value (DV), which preferably is a difference between the two parameters (P) or a quotient of the two parameters (P).

**5.** Data processing apparatus (12) according to one of the preceding claims, **characterised in that**
the data processing apparatus (12) is configured to calculate a normalised T-wave deviation value (N 1 DV, N2TDV, N2LDV) or a normalised ST-segment deviation value by relating the T-wave deviation value (1 DV, 2TDV, 2LDV) or the ST-segment deviation value which is based on a parameter (P) of T-waves (T) and/or ST-segments (ST) to a T-wave characteristic value (CV) or a ST-segment characteristic value of the same parameter (P) from the same heart, and particularly from the same source of T-waves (T) or ST-segments (ST) as the the T-wave deviation value (1 DV, 2TDV, 2LDV) or the ST-segment deviation value, and more particularly from the same lead as the T-wave deviation value (1DV, 2TDV, 2LDV) or the ST-segment deviation value.

**6.** Data processing apparatus (12) according to one of the preceding claims, **characterised in that** the apparatus is configured
to compare a T-wave deviation value (1 DV) or a normalised T-wave deviation value (N1 DV) or a ST-segment deviation value or a normalised ST-segment deviation value from a first source representing a first location at the heart to a T-wave deviation value (1 DV) or a normalised T-wave deviation value (N1DV) or a ST-segment deviation value or a normalised ST-segment deviation value, from a second source representing a second location at the heart that is different from the first location and
to generate a second-order local deviation value (2LDV) or a normalised second-order local deviation value (N2LDV).

**7.** Data processing apparatus (12) according to one of the preceding claims, **characterised in that** the apparatus is configured to
process a number of at least 3 T-waves (T) and/or ST-segments (ST), preferably at least 60 T-waves (T) and/or ST-segments (ST) and most preferably about 200 T-waves (T) or ST-segments (ST) and/or to process a number of T-waves (T) and/or
a number of ST-segments (ST) of a PQRST-complex during at least 1 second, preferably at least 2, 3 or 4 seconds, more preferably at least 30 seconds and most preferably about 200 seconds to obtain T-wave deviation values (1 DV) or normalised T-wave deviation values (N1 DV) or ST-segment deviation values or normalised ST-segment deviation values and to preferably calculate a second order deviation value (2LDV, 2TDV) or a normalised second order deviation value (N2LDV, N2TDV) from the obtained T-wave deviation values (1DV) or normalised T-wave deviation values (N1 DV) or ST-segment deviation values or normalised ST-segment deviation values.

**8.** Data processing apparatus (12) according to one of the preceding claims, **characterised in that** the apparatus is configured
to compare a first T-wave deviation value (1 DV) or a first normalised T-wave deviation value (N1 DV) or a first ST-segment deviation value or a first normalised ST-segment deviation value measured at a first time
to a second T-wave deviation value (1 DV) or a second normalised T-wave deviation value (N1DV) or a second ST-segment deviation value or a second normalised ST-segment deviation value measured at a second time that is different from the first time and
to generate a second order time deviation value (2TDV) or a normalised second order time deviation value (N2TDV) which is based on the comparison.

9. Data processing apparatus (12) according to one of the preceding claims, **characterised in that** the data processing apparatus comprises a measurement device for generating T-wave information (TI) or ST-segment information from an individual human or animal by measurement,
wherein the measurement device preferably has a resolution of less than 5ms, preferably less than 285µs, preferably less than 100µs and most preferable less than 50µs in time and of less than 50mV and preferably less than 16µV in amplitude, preferably less than 1µV and most preferable less than 1 nV.

10. Data processing apparatus (12) according to one of the preceding claims, **characterised in that** the data processing apparatus (12) comprises a value signalling means which is configured to signal and/or display a deviation value and/or a normalised deviation value of a parameter and/or a second order deviation value and/or a second order normalised deviation value to a person or to transfer corresponding data to another unit as the data processing apparatus.

11. Assessment apparatus (14) for an assessment of a condition or a disease of at least a part of a myocardium of a heart of an individual human or animal, **characterised in that** the assessment apparatus (14) is configured to base an assessment or a diagnosis on at least one T-wave deviation value and/or normalised T-wave deviation value and/or a ST-segment deviation value and/or normalised ST-segment deviation value and/or a second order deviation value provided by a data processing apparatus according to one of the claims 1 to 10 to assess or diagnose a condition of at least a part of a myocardium and preferably of a heart.

12. Assessment apparatus (14) according to claim 11, **characterised in that** the apparatus is configured to assess

   A) risk and/or presence and/or extent of ischemic heart disease by a T-wave deviation value (1 DV) or a normalised T-wave deviation value (N1 DV) or a ST-segment deviation value or a normalised ST-segment deviation value of parameter time period, and/or by a T-wave deviation value (1 DV) or a normalised T-wave deviation value (N1 DV) or a ST-segment deviation value or a normalised ST-segment deviation value of parameter amplitude, and/or
   B) risk and/or presence and/or extent of stenosis
   by a T-wave deviation value (1 DV) or a normalised T-wave deviation value (N1 DV) and/or a ST-segment deviation value or a normalised ST-segment deviation value of parameter time period, and/or
   C) risk and/or presence and/or extent of angina pectoris caused by a heart condition or disease
   by a T-wave deviation value (1 DV) or a normalised T-wave deviation value (N1 DV) or a ST-segment deviation value or a normalised ST-segment deviation value of parameter time period and a T-wave deviation value (1 DV) or a normalised T-wave deviation value (N1 DV) or a ST-segment deviation value or a normalised ST-segment deviation value of parameter amplitude or an area parameter or one or more function parameters, and/or
   D) risk and/or presence and/or extent of syndrome X
   by a T-wave deviation value (1 DV) or a normalised T-wave deviation value (N1 DV) or a ST-segment deviation value or a normalised ST-segment deviation value of parameter time period and a deviation value of parameter amplitude or an area parameter or one or more function parameters, and/or
   E) risk and/or presence and/or extent of myocardial infarction, especially acute myocardial infarction,
   by a T-wave deviation value (1DV) or a normalised T-wave deviation value (N1DV) or a ST-segment deviation value or a normalised ST-segment deviation value of parameter time period, and/or by a quotient of a a T-wave deviation value (1 DV) or a normalised T-wave deviation value (N1 DV) or a ST-segment deviation value or a normalised ST-segment deviation value of parameter time period and a T-wave deviation value (1 DV) or a normalised T-wave deviation value (N 1 DV) or a ST-segment deviation value or a normalised ST-segment deviation value of parameter amplitude, and/or
   F) risk and/or presence and/or extent of sudden heart death
   by a T-wave deviation value (1 DV) or a normalised T-wave deviation value (N 1 DV) or a ST-segment deviation value or a normalised ST-segment deviation value of parameter amplitude, and/or
   G) a NYHA classification
   by a T-wave deviation value (1 DV) or a normalised T-wave deviation value (N1 DV) or a ST-segment deviation value or a normalised ST-segment deviation value with parameter amplitude, and/or
   H) an event time period or an event point in time for a condition or a disease of the heart as mentioned in item A) to G)

by a second order time deviation value (2TDV) or a normalised second order time deviation value (N2TDV) of a parameter as mentioned under A) to G),

in respect of a certain condition or disease,

particularly an event time period to an acute myocardial infarction or an event point in time of an acute myocardial infarction

by a second order time deviation value (2TDV) or a normalised second order time deviation value (N2TDV) of parameter time period or by a second order time deviation based on a quotient of parameters time period and parameter amplitude, , and/or

I) a location at the heart of a condition or a disease of the heart as mentioned as item A) to G) by a second order local deviation value (2LDV) or a second order local deviation value (N2DLV) of a parameter mentioned under A) toG) for the condition or disease, respectively,

wherein preferably the assessment apparatus (14) comprises an assessment signalling means for signalling an assessment of a condition or disease according to at least one of the items A) to G) or data generated according to items H) or I) to a person or to transfer corresponding data to another unit as the data processing apparatus.

13. Assessment apparatus (14) according to claim 12, **characterised in that** the apparatus is configured to carry out a comparison of assessment information to a threshold value in order to make a diagnosis proposal and/or to make an automatic diagnosis and/or to automatically notify a medical service when a decision about a predefined risk, presence or extent of condition or disease an acute myocardial infarction has been made,

wherein preferably the assessment apparatus (14) comprises a diagnosis signalling means for signalling a diagnosis proposal or an automatic diagnosis of a condition or disease according to at least one of the items A) to G) to a person or to transfer corresponding data to another unit as the data processing apparatus.

14. Method for providing data for an assessment and/or a diagnosis of a condition or a disease of at least a part of the myocardium or the whole myocardium of a heart of a human or an animal,

wherein the method includes processing T-wave information (TI) or ST-segment information from a signal from a myocardium of a heart of a human or animal,

**characterised in that** the method includes deriving a T-wave deviation value (1 DV) or a ST-segment deviation value from differences of at least two T-wave (T) or ST-segment parameters (P) of the same type which originate from different heartbeats of the heart in order to generate assessment data (22).

15. Method for assessing a condition or a disease of the heart of a human or animal, **characterised in that** least one of the following assessments is carried out with first and/or second order deviation values and/or first and/or second order normalised deviation values which are provided by a data processing apparatus according to claims 1 to 10 and/or by a method for providing data according to claim 14:

A) risk and/or presence and/or extent of ischemic heart disease by a T-wave deviation value (1 DV) or a normalised T-wave deviation value (N1 DV) or a ST-segment deviation value or a normalised ST-segment deviation value of parameter time period, and/or by a T-wave deviation value (1 DV) or a normalised T-wave deviation value (N1 DV) or a ST-segment deviation value or a normalised ST-segment deviation value of parameter amplitude, and/or

B) risk and/or presence and/or extent of stenosis

by a T-wave deviation value (1DV) or a normalised T-wave deviation value (N1DV) and/or a ST-segment deviation value or a normalised ST-segment deviation value of parameter time period, and/or

C) risk and/or presence and/or extent of angina pectoris caused by a heart condition or disease

by a T-wave deviation value (1DV) or a normalised T-wave deviation value (N1DV) or a ST-segment deviation value or a normalised ST-segment deviation value of parameter time period and a T-wave deviation value (1 DV) or a normalised T-wave deviation value (N1 DV) or a ST-segment deviation value or a normalised ST-segment deviation value of parameter amplitude or an area parameter or one or more function parameters, and/or

D) risk and/or presence and/or extent of syndrome X

by a T-wave deviation value (1 DV) or a normalised T-wave deviation value (N1 DV) or a ST-segment deviation value or a normalised ST-segment deviation value of parameter time period and a deviation value of parameter amplitude or an area parameter or one or more function parameters, and/or

E) risk and/or presence and/or extent of myocardial infarction, especially acute myocardial infarction,

by a T-wave deviation value (1DV) or a normalised T-wave deviation value (N1DV) or a ST-segment deviation value or a normalised ST-segment deviation value of parameter time period, and/or by a quotient of a a T-wave deviation value (1 DV) or a normalised T-wave deviation value (N 1 DV) or a ST-segment deviation value or a normalised ST-segment deviation value of parameter time period and a T-wave deviation value (1 DV) or a normalised T-wave deviation value (N1 DV) or a ST-segment deviation value or a normalised ST-segment deviation value of parameter amplitude, and/or

F) risk and/or presence and/or extent of sudden heart death

by a T-wave deviation value (1 DV) or a normalised T-wave deviation value (N1 DV) or a ST-segment deviation value or a normalised ST-segment deviation value of parameter amplitude, and/or

G) a NYHA classification

by a T-wave deviation value (1DV) or a normalised T-wave deviation value (N1DV) or a ST-segment deviation value or a normalised ST-segment deviation value with parameter amplitude, and/or

H) an event time period or an event point in time for a condition or a disease of the heart as mentioned in item A) to G)

by a second order time deviation value (2TDV) or a normalised second order time deviation value (N2TDV) of a parameter as mentioned under A) to G),

in respect of a certain condition or disease,

particularly an event time period to an acute myocardial infarction or an event point in time of an acute myocardial infarction

by a second order time deviation value (2TDV) or a normalised second order time deviation value (N2TDV) of parameter time period or by a second order time deviation based on a quotient of parameters time period and parameter amplitude, , and/or

I) a location at the heart of a condition or a disease of the heart as mentioned as item A) to G) by a second order local deviation value (2LDV) or a second order local deviation value (N2DLV) of a parameter mentioned under A) toG) for the condition or disease, respectively,

wherein preferably condition information and/or disease information is generated according to at least one of the items A) to I) and signalled and/or displayed to a person as a condition information and/or disease information and/or is transferred to another unit, preferably to a medical service or a diagnosis means.

16. Method according to claim 15, **characterised in that** a comparison of assessment data (22) with at least one threshold value is made

by a diagnosis device

in order to generate a diagnosis proposal and/or

in order to generate an automatic diagnosis and/or

by a human that makes a diagnosis decision,

wherein preferably diagnosis information is generated and signalled and/or displayed to a person as a diagnosis proposal and/or as an automatic diagnosis, and/or the diagnosis information is transferred to another unit, preferably to a medical service.

Fig. 1

Fig. 2

TP

T

AM

Z

AR

F

## Fig. 3

Measurement data — 21

↓

Data processing apparatus — 12

↓

Assessment data — 22

Assessment data — 22

↓

Assessment apparatus — 14

↓

Condition information — 23

## Fig. 4

## Fig. 5

Parameters of T-wave — *P*

*TP*

Time period (TP) of T-wave

Lactic acid (L) metabolism — *L*

TP correlated with
- Stenosis
- Danger of acute
  myocardial infarction

*AM*

Amplitude (AM) of T-wave

Glucose (G) metabolism — *G*

TP and AM correlated with
- Angina pectoris
- Syndrome X

AM correlated with
- Sudden heart death
- NYHA-class

*AR*

Area (AR) bordered by T-wave

Anaerobic metabolism — *A*

Common value for
glucose (G) metabolism and
lactic acid (L) metabolism

AR Correlated with
- Angina pectoris
- Syndrome X

*F*

Function parameter (F) of
T-wave approximating function

Anaerobic metabolism — *A*

Common value for
glucose (G) metabolism and
lactic acid (L) metabolism

When TP and AM
or AR derived from F:
 Correlated with
- Angina pectoris
- Syndrome X

When TP
derived from F:
 Correlated with
- Stenosis
- Danger of acute
  myocardial infarction

When AM
derived from F:
 Correlated with
- Sudden heart death
- NYHA-class

Fig. 6

Metabolism of Myocardium

electrical processes

T-Wave
First in time — *T*

T-Wave
Second in time — *T*

measurement

measurement

T-wave information — *TI*

T-wave information — *TI*

extraction,
calculation
of parameter

extraction,
calculation
of parameter

Parameter of T-wave
First in time — *P*

Parameter of T-wave
Second in time — *P*

Further
parameters of T-wave
of same type — *P*

calculation of
a difference

calculation of
a mean value
or the like

Non-normalised
first order deviation value — *1DV*

Dividend

T-wave characteristic
value of parameter

Quotient: Normalised
first order deviation value

Divisor

*CV*

*N1DV*

# Fig. 7

$1DV, N1DV$
$1DV, N1DV$

First measurement time                    Second measurement time

| First non-normalised or normalised first order T-wave deviation value | Second non-normalised or normalised first order T-wave deviation value |

calculation

| For example difference, quotient, differentiation |

| Trend in time Non-normalised or normalised second order time deviation value | ── $2TDV, N2TDV$

## Fig. 8

$1DV, N1DV$

Frist measurement location                     Second measurement location
from an individual          $1DV,$          from the same individual
                            $N1DV$

| First First non-normalised or normalised first order T-wave deviation value | Second non-normalised or normalised first order T-wave deviation value |

calculation

| Difference or quotient |

| Difference or relation between locations Non-normalised or normalised second order local deviation value |

## Fig. 9

$2LDV, N2LDV$

Europäisches
Patentamt

European
Patent Office

Office européen
des brevets

# EUROPEAN SEARCH REPORT

**Application Number**

EP 16 00 0336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 8 521 267 B1 (JALALI LALEH [US] ET AL) 27 August 2013 (2013-08-27)<br><br>* column 2, line 11 *<br>* column 5, line 4 *<br>* column 6, line 38 *<br>* column 9, lines 55, 57, 58, 63-67 *<br>* column 10, line 1 *<br>* figures 2-4, 7 * | 1,3,4,7, 8,10, 12-16 | INV.<br>A61B5/04<br>A61B5/0452<br>A61B5/02<br>A61B5/00<br><br>ADD.<br>A61B5/0408<br>G06F19/00 |
| X | WO 2005/096925 A2 (QUINTON CARDIOLOGY INC [US]; HADLEY DAVID [US]; SAGIROGLU MUSTAFA [US]) 20 October 2005 (2005-10-20)<br>* paragraphs [0004] - [0007], [0009], [0013], [0045], [0046], [0067], [0073] *<br><br>* figure 16 * | 1-4,6-16 | |
| X | US 7 254 440 B1 (KROLL MARK W [US]) 7 August 2007 (2007-08-07)<br>* column 2, lines 1-50 *<br>* column 3, lines 15-24 *<br>* column 8, lines 9, 14, 16-20 *<br>* column 9, line 15 *<br>* figures 2, 5 * | 1,3-5, 10,12-16 | |
| | | | TECHNICAL FIELDS SEARCHED (IPC) |
| | | | A61B<br>G06F |
| A | US 2009/281441 A1 (ZHANG HONGXUAN [US] ET AL) 12 November 2009 (2009-11-12)<br>* the whole document * | 1-16 | |
| A | US 2013/274623 A1 (ZHANG HONGXUAN [US]) 17 October 2013 (2013-10-17)<br>* the whole document * | 1-16 | |
| A | WO 2009/091583 A1 (MASSACHUSETTS INST TECHNOLOGY [US]; GUTTAG JOHN V [US]; SYED ZEESHAN H) 23 July 2009 (2009-07-23)<br>* the whole document * | 1-16 | |

-/--

The present search report has been drawn up for all claims

| Place of search | Date of completion of the search | Examiner |
|---|---|---|
| The Hague | 30 November 2016 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 16 00 0336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2004/215090 A1 (ERKKILA JOUNI [FI] ET AL) 28 October 2004 (2004-10-28) * the whole document * ----- | 1-16 | |
| A | US 2007/249944 A1 (FISCHELL DAVID R [US] ET AL) 25 October 2007 (2007-10-25) * the whole document * ----- | 1-16 | |
| A | US 4 947 857 A (ALBERT DAVID E [US] ET AL) 14 August 1990 (1990-08-14) * the whole document * ----- | 1-16 | |

**TECHNICAL FIELDS SEARCHED (IPC)**

The present search report has been drawn up for all claims

| Place of search | Date of completion of the search | Examiner |
|---|---|---|
| The Hague | 30 November 2016 | Meyer, Wolfgang |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                         
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 00 0336

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2016

| Patent document cited in search report | | Publication date | Patent family member(s) | | Publication date |
|---|---|---|---|---|---|
| US 8521267 | B1 | 27-08-2013 | NONE | | |
| WO 2005096925 | A2 | 20-10-2005 | US<br>US<br>US<br>US<br>WO | 2005222510 A1<br>2005222511 A1<br>2005222512 A1<br>2005222513 A1<br>2005096925 A2 | 06-10-2005<br>06-10-2005<br>06-10-2005<br>06-10-2005<br>20-10-2005 |
| US 7254440 | B1 | 07-08-2007 | NONE | | |
| US 2009281441 | A1 | 12-11-2009 | NONE | | |
| US 2013274623 | A1 | 17-10-2013 | NONE | | |
| WO 2009091583 | A1 | 23-07-2009 | US<br>US<br>US<br>WO | 2009192394 A1<br>2013046193 A1<br>2014296724 A1<br>2009091583 A1 | 30-07-2009<br>21-02-2013<br>02-10-2014<br>23-07-2009 |
| US 2004215090 | A1 | 28-10-2004 | NONE | | |
| US 2007249944 | A1 | 25-10-2007 | US<br>US<br>US<br>US<br>US<br>US | 2007249944 A1<br>2007293775 A1<br>2007293778 A1<br>2014107454 A1<br>2015018658 A1<br>2016128595 A1 | 25-10-2007<br>20-12-2007<br>20-12-2007<br>17-04-2014<br>15-01-2015<br>12-05-2016 |
| US 4947857 | A | 14-08-1990 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALEKSEYEV, MICHAEL ; ALEKSEYEV ALEKSANDR ; DOWZHIKOV, ANDREW ; LABIN, SERGEI.** Digital Analytic Cardiography (DACG), a New Method for Quantitiative Trophism Assessment of the Myorcardium. *International Journal of Cardio-vascular Research,* 2014, vol. 3, 6